# EUROPEAN PATENT SPECIFICATION

(11) **EP 2 164 520 B1**
(45) Date of publication and mention of the grant of the patent: **30.10.2019**
(21) Application number: 08762219.7
(22) Date of filing: 02.06.2008
(51) Int. Cl.: A61K 47/69, A61K 9/20, C08B 37/16, A61P 19/00, A61P 9/00, B82Y 5/00

(54) **Cyclodextrins for administering fatty acids in tablets**
Cyclodextrine für die Verabreichung von Fettsaüren in Tabletform
Des cyclodextrines pour administrer des acides gras sous forme de comprimés.

(30) Priority: 31.05.2007 GB 0710439
(43) Date of publication of application: 24.03.2010
(73) Proprietor: Golden Omega Norway AS, 0422 Oslo (NO)
(72) Inventor: KLAVENESS, Jo, 1166 Oslo (NO); BRUDELI, Bjarne, 0663 Oslo (NO); RONGVED, Pal, 0173 Oslo (NO)
(74) Representative: Dehns
(86) International application number: PCT/GB2008/001861
(87) International publication number: WO 2008/146016

(56) References cited:
- EP-A- 0 657 176
- WO-A-2008/083213
- FR-A- 2 790 758
- GB-A- 2 104 907
- GB-A- 2 146 650
- KOBAYASHI KOJI ET AL: "The effect of n - 3 PUFA/gamma-cyclodextrin complex on serum lipids in healthy volunteers--a randomized, placebo-controlled, double-blind trial" ASIA PACIFIC JOURNAL OF CLINICAL NUTRITION, vol. 16, no. 3, 1 January 2007 (2007-01-01), pages 429-434, XP002515873 ISSN: 0964-7058
- FENG GUANGZHU ET AL: "Preparation of inclusion compound of .beta.- cyclodextrin with eicosapentaenoic acid (EPA) and its property of the thermal decomposition kinetics" ZHENGZHOU GONGCHENG XUEYUAN XUEBAO, vol. 25, no. 3, 2004, pages 44-48, XP008106417 ISSN: 1671-1629
- YOSHII, H. ET AL: "Oxidation stability of eicosapentaenoic and docosahexaenoic acid included in cyclodextrins" JOURNAL OF INCLUSION PHENOMENA AND MOLECULAR RECOGNITION IN CHEMISTRY, vol. 25, no. 1-3, 1996, pages 217-220, XP002528788 ISSN: 0923-0750
- VON SCHACKY ET AL: "Cardiovascular benefits of omega-3 fatty acids" CARDIOVASCULAR RESEARCH, vol. 73, no. 2, 21 December 2006 (2006-12-21), pages 310-315, XP005835248 ISSN: 0008-6363
- T.L. VESTLAND ETAL.: "Bioavailability of Eicosapentaenoic Acid and Docosahexaenoic Acid from Omega-3 Tablets", JSM BIOAVAILABILITY AND BIOEQUIVALENCE, vol. 1, 5 February 2017 (2017-02-05), pages 1001-1-1001-5,
- VESTLAND TINA LIEN ET AL: "Characterization of omega-3 tablets", FOOD CHEMISTRY, ELSEVIER LTD, NL, vol. 197, 30 October 2015 (2015-10-30), pages 496-502, XP029312120, ISSN: 0308-8146, DOI: 10.1016/J.FOODCHEM.2015.10.142
- VESTLAND TINA LIEN ET AL: "Compactible powders of omega-3 and [beta]-cyclodex", FOOD CHEMISTRY, ELSEVIER LTD, NL, vol. 185, 4 April 2015 (2015-04-04), pages 151-158, XP029156396, ISSN: 0308-8146, DOI: 10.1016/J.FOODCHEM.2015.03.132

## Description

This invention relates to pharmaceutical and nutraceutical products in the form of tablets comprising at least two fatty acid triglycerides and at least one cyclodextrin optionally together with vitamins, minerals and/or pharmaceuticals, and the use of such tablets in treatment or prophylaxis of disorders related to the cardiovascular system, skin and bone. In particular, the invention concerns the use of tablets comprising high concentrations and high doses of omega-3 fatty acids or derivatives thereof in the treatment or prevention of hypertriglyceridemia and cardiac infection.

Omega-3 and omega-6 fatty acids are fatty acids essential to human health but ones which cannot be manufactured by the body. For this reason, omega-3 fatty acids must be obtained from food sources and can be found in fish and certain plant oils. It is important to maintain an appropriate balance of omega-3 and omega-6 (another essential fatty acid) in the diet as these two substances work together to promote health. Omega-3 and omega-6 fatty acids play a crucial role in brain function as well as normal growth and development for example.

There are three major types of unsaturated fatty acids that are ingested in foods and used by the body: the omega-6 fatty acid alpha-linolenic acid (ALA), and the omega-3 fatty acids eicosapentaenoic acid (EPA) and docosahexaenoic acid (DHA). Once eaten, the body converts ALA to EPA and DHA, the two types of omega-3 fatty acids more readily used by the body. Extensive research indicates that omega-3 fatty acids reduce inflammation and help prevent certain chronic diseases such as heart disease and arthritis. These essential fatty acids are highly concentrated in the brain and appear to be particularly important for cognitive and behavioural function. In fact, infants who do not get enough omega-3 fatty acids from their mothers during pregnancy are at risk for developing vision and nerve problems.

As mentioned previously, it is very important to maintain a balance between omega-3 and omega-6 fatty acids in the diet. For example, omega-3 fatty acids help reduce inflammation whereas most omega-6 fatty acids tend to promote inflammation. An inappropriate balance of these essential fatty acids contributes to the development of disease while a proper balance helps maintain and even improve health. A healthy diet should consist of roughly one to four times more omega-6 fatty acids than omega-3 fatty acids.

With the development of convenience foods and a general decline in the consumption of healthy foodstuffs such as fresh fish, fruit and vegetables, the typical American diet tends to contain 11 to 30 times more omega-6 fatty acids than omega-3 fatty acids and many researchers believe this imbalance is a significant factor in the rising rate of inflammatory disorders in the United States.

In contrast, however, the Mediterranean diet consists of a healthier balance between omega-3 and omega-6 fatty acids and many studies have shown that people who follow this diet are less likely to develop heart disease. The Mediterranean diet does not include much meat (which is high in omega-6 fatty acids) and emphasizes foods rich in omega-3 fatty acids including whole grains, fresh fruits and vegetables, fish, olive oil, garlic, as well as moderate wine consumption.

Thus, since their discovery in the 1970s, and the finding that the ratio of omega-3 to omega-6 acids is imbalanced in the diets of many individuals, omega-3 fatty acids or their derivatives have been made available to consumers as dietary supplements to try to restore the desired omega-3 to omega-6 balance. Omega-3 fatty acids or derivatives thereof are thus now taken routinely by many hundreds of thousands of individuals to prevent a variety of illnesses such as arthritis, cardiac infarction and stroke.

Omega-3 fatty acids are often provided to consumers in their naturally occurring triglyceride form. The Omega-3 fatty acid triglyceride or the free fatty acid itself are generally sourced from natural oils such as marine oils. Since it is difficult to isolate the omega-3 acids in high purity from marine oils, omega-3 supplements often possess an unpleasant fishy after taste which the consumer dislikes. It is also a major problem for many individuals, such as the elderly and children, to swallow the gelatine capsules used today to contain the omega-3 material. Capsules are also expensive to prepare. It would be useful therefore if omega-3 compounds could be offered in alternative dosage forms.

A further problem with unsaturated fatty acids is their stability. Due to the presence of the double bonds in the fatty acid backbone, these materials are readily oxidised and go rancid. Typical shelf life for an omega-3 based pharmaceutical product in soft gelatin capsules is about 3 years (Omecor® from Pronova/Pfizer). The skilled man has therefore been looking at ways of preventing oxidation.

One solution to the stability problem has been to complex the polyunsaturated fatty acids with a compound such as cyclodextrin. EP-A-470452 (Staroil) describes methods for the preparation of polyunsaturated fatty acid complexes with cyclodextrins in aqueous solution.

US 4,438,106 (Kureaha) describes preparation of cyclodextrin EPA and DHA complexes. These complexes are prepared using large excess of cyclodextrins.

WO00/53637 (Commissariat a L energie atomique) describes fatty acid complexes with gamma-cyclodextrin.

GB2146650 (Hayashibara Biochem Lab) describes inclusion compounds of EPA and cyclodextrin.

GB2104907 describes inclusion compounds of EPA and DHA with cyclodextrins.

Guang-zhu et al (Zhengzhou Gongcheng Xueyuan Xuebao, Vol. 25, no. 3, 2004, 44-48) describes the inclusion of EPA with beta cyclodextrin.

Yoshii et al. (Journal of Inclusion Phenomena and Molecular Recognition in Chemistry 25: 217-220, 1996) describes the oxidation stability of EPA and DHA included in cyclodextrins.

Fatty acid complexes between fatty acids and derivatives thereof with cyclodextrins are thus described in the prior art, however, the weight ratio between cyclodextrin and fatty acid is high, typically around 3 and higher. These prior art disclosures thus require a considerable amount of cyclodextrin to be present and hence a limited amount of the desired fatty acid material. Since the amount of fatty acid required in order to attain a pharmacologically active quantity of active ingredient is high, this means that regular doses of the fatty acid need to be administered.

This problem is further exacerbated when the complex with cyclodextrin is formulated into a dosage form. As the dosage form necessarily contains components other than the fatty acid complex (i.e. excipients etc) the amount of actual fatty acid material in the dosage form can be quite low. It is not uncommon therefore for oversized dosage forms to be used simply to allow administration of sufficient material over the course of a day.

The current solution to the problems of omega-3 fatty acid formulation is therefore the use of a capsule which contains a considerable amount of oily liquid and therefore delivers a sufficient dose. As noted above however, capsules are expensive, leave a bad "fishy" taste in the mouth of the consumer and are susceptible to oxidation.

The most popular oral dosage form is a tablet, and it would be advantageous if a fatty acid containing tablet could be made available which does not suffer from the problems of taste, oxidation, expense and the need for multiple/oversize dosage forms.

Tablet dosage forms of fatty acids such as DHA and EPA are known. US 5843919 (Burger) describes mixtures of glucosamine and EPA. This tablet is however, free of cyclodextrin and uses the specific particulate compound EPA to formulate the tablet. No consideration is given to tableting where the fatty acid component is in the form of an oil.

EP342795 (Taiyo) describes compositions for improving cerebral function which contain DHA. Tablets are mentioned as a possible dosage form. No consideration is given to tableting where the fatty acid component is in the form of an oil and again these compositions are free of cyclodextrin.

WO88/02221 (Kabivitrum) describes an EPA/DHA granulate for tablet preparation using sugar carriers.

US 4831022 (Hijiya) describes EPA inclusion complexes with gamma cyclodextrins with up to 47.6% EPA in the solid prepared. This invention uses a pure free fatty acid rather than an oily mixture thereof. No consideration is given to tableting where the fatty acid component is in the form of an oil.

Whilst tablets comprising fatty acids and derivatives thereof, including tablets comprising cyclodextrin fatty acid complexes, are described in prior art, all these tablets comprise relative low amounts of fatty acids, comprise only the expensive compound EPA and not other omega-3 fatty acids and are formulated using particulate fatty acid compounds starting materials.

As noted above, fatty acids and derivatives thereof when used as pharmaceuticals and nutraceuticals must be present in relative high doses. A typical dose of fatty acids can be from about one to several grams per day . Prior art tablet formulations comprising small amounts of fatty acids and derivatives thereof are therefore not useful formulations for human use. Although such formulations have been known in the art for several years, the market is still saturated with oil based dosage forms such as capsules comprising oils.

Fatty acid based products are used in high doses all over the world both as pharmaceutical products and nutraceutical products, and there is a need for new cheap and stable one-dose formulations, especially tablets, comprising a high dose of fatty acid or derivatives thereof.

Surprisingly, we have now found that fatty acid triglycerides in the form of complexes with cyclodextrins prepared as stable solid materials, can easily be transformed into tablets with very high concentration of the fatty acid compound. The present inventors have realised that ideal dosage forms for these compounds are tablets and these are readily swallowed and are cheap to manufacture. In particular, the inventors have found that tablets containing complexes of fatty acid triglycerides with cyclodextrin can be prepared by direct compression and moreover they can be prepared having a very high concentration of the desired active agent.

Thus, viewed from one aspect the invention provides a pharmaceutical or nutraceutical tablet for oral administration comprising at least 20 wt% of solid complex formed between at least two fatty acid triglycerides and at least one cyclodextrin, wherein the content of said at least two fatty acid triglycerides in said complex is 10 wt% or more, wherein the weight ratio of fatty acid compounds (total) to cyclodextrin is 1:5 to 5:1 and wherein the cyclodextrin is a beta-cyclodextrin.

Viewed from another aspect the invention provides a pharmaceutical or nutraceutical tablet for oral administration comprising a complex formed between at least one fatty acid or derivative thereof and at least one cyclodextrin wherein the at least one fatty acid or derivative thereof is in the form of an oil when combined with the cyclodextrin.

Viewed from another aspect the invention provides a pharmaceutical or nutraceutical tablet for oral administration as described above comprising more than 25 wt%, especially more than 50 wt% of a solid complex formed between at least two fatty acid triglycerides and at least one cyclodextrin.

Viewed from another aspect the invention provides a pharmaceutical or nutraceutical tablet for oral administration as described above comprising more than 170 mg, e.g. at least 300 mg of at least two fatty triglycerides and at least one cyclodextrin.

Viewed from another aspect the invention provides a pharmaceutical or nutraceutical tablet for oral administration as described above comprising an omega-3 fatty acid or derivative thereof and beta-cyclodextrin.

Viewed from another aspect the invention provides a pharmaceutical or nutraceutical tablet for oral administration as described above comprising DHA and EPA triglycerides or derivatives thereof and beta-cyclodextrin.

Viewed from another aspect the invention provides a pharmaceutical or nutraceutical tablet for oral administration as described above comprising EPA and/or DHA triglycerides and calcium carbonate.

Viewed from another aspect the invention provides a pharmaceutical or nutraceutical tablet for oral administration as described above further comprising a calcium salt.

Viewed from another aspect the invention provides a pharmaceutical or nutraceutical tablet for oral administration as described above comprising a complex formed between at least two fatty acid triglycerides, at least one cyclodextrin and simvastatin.

Viewed from another aspect the invention provides a pharmaceutical or nutraceutical tablet for oral administration as described above comprising a complex formed between at least two fatty acid triglycerides, at least one cyclodextrin and atorvastatin.

Viewed from another aspect the invention provides a pharmaceutical or nutraceutical tablet for oral administration as described above comprising a complex formed between at least two fatty acid triglycerides, at least one cyclodextrin and glucosamine.

Viewed from another aspect the invention provides a pharmaceutical or nutraceutical tablet for oral administration as described above comprising a complex formed between at least two fatty acid triglycerides, at least one cyclodextrin and at least one vitamin.

Viewed from another aspect the invention provides a pharmaceutical or nutraceutical tablet for oral administration as described above comprising a complex formed between at least two fatty acid triglycerides, at least one cyclodextrin and folic acid.

Viewed from another aspect the invention provides a pharmaceutical tablet as hereinbefore described for use in the treatment and prophylactic treatment of diseases of the cardiovascular system and bone.

Viewed from another aspect the invention provides a method for the manufacture of a tablet as hereinbefore defined by direct compression.

By nutraceutical is any substance that is a food or a part of a food and provides medical or health benefits, including the prevention and treatment of disease.

By derivative of a fatty acid, e.g. omega-3 or omega-6 fatty acid, is meant a salt, amide or ester thereof, or any other compound where the COOH group is functionalised in such a way that it will return to a COOH group upon treatment, e.g. upon hydrolysis, e.g. a phospholipid thereof. The fatty acid compounds in the tablets of the invention are in the form of triglycerides, i.e. the fatty acid derivative is a triglyceride. Preferred salts are those of alkali metals, e.g. sodium or ammonium salts, in particular polyamino alcohol salts. Mixtures of derivatives and/or acids may be present.

In the description which follows, the term "fatty acid compound" is used to cover a fatty acid *per se* or a derivative thereof.

The at least one fatty acid or derivative thereof present in the tablets of the invention is preferably unsaturated, especially polyunsaturated. Most preferably, the tablets of the invention comprise at least one omega-3 fatty acid compound.

Any fatty acid compound, preferably an omega-3 fatty acid compound, present in the tablet can preferably be synthetic or semisynthetic but preferably it is derived from a natural source such as a plant oil or an animal oil. Oils which contain fatty acids, typically present as esters of the fatty acids, are well known in the art. Suitable plant oils include rapeseed oil, corn oil, soya oil, sunflower oil, vegetable oil and olive oil. Preferably however, the natural source of the fatty acid is an animal oil such as tallow oil.

Highly preferably, the source of the fatty acid compound is a marine oil, such as a fish oil or krill oil. Crude marine oil used in this invention can be derived from any marine source such as fish, especially seawater fish such as tuna, sardines, salmon, mackerel, herring, trout, halibut, cod, haddock, catfish, sole etc. The use of oily fish is preferred. Ideally however, the crude marine oil will derive from marine mammals such as seals, walrus or sea lions, preferably seals or from krill. Seal oil has been found to be especially rich in omega-3 fatty acid compounds, e.g. of the order of 20-25 wt% and therefore forms an ideal starting material to form the tablets of the invention. Seal oils are available from a variety of commercial sources.

The tablet contains at least two fatty acid triglycerides. Preferably, it contains a mixture of fatty acid compounds, especially unsaturated fatty acid compounds, especially a mixture of polyunsaturated fatty acid compounds. It will be appreciated that the tablets of the invention might also contain saturated fatty acid compounds as these are also present in naturally occurring unsaturated fatty acid compound sources.

An unsaturated fatty acid compound contains one or more carbon carbon double bonds in the carbon backbone. Preferably, the carbon backbone is polyunsaturated. Preferably, at least one fatty acid is an omega-3 fatty acid compound in which the double bond most distant from the carboxylic acid functionality is located at the third bond counted from the end (omega) of the carbon chain. The fatty acid compound may also be an omega-6 fatty acid compound where the double bond most distant from the carboxylic acid functionality is located at the sixth bond counted from the end (omega) of the carbon chain. A tablet of the invention most preferably contains a variety of omega-3 and omega-6 fatty acid compounds.

The total concentration of omega-3 fatty acid compounds in a crude oil varies depending on the natural source in question but, for example, in sea fish, the amount of the omega-3 compounds is approximately 25 wt%.

Unsaturated fatty acid compounds which can form part of the tablet of the invention may be those of formula (I):

CH₃(CH₂)ₙ-(CH=CH-CH₂)m-(CH₂)ₛ-COOH (I)

wherein n, m and s are integers, e.g. of 1 to 10;
or a derivative thereof.

Subscript n is preferably 1. Subscript m is preferably 2 to 8. Subscript s is preferably 1 to 6. Ideally, the carbon chain is linear although it is within the scope of the invention for the backbone to carry alkyl side chains such as methyl or ethyl. (For this formula DHA n=1, m=6 and s=1, for EPA n=1, m=5 and s=1. In ALA, n=4, m=2 and s=6) .

Omega-3 fatty acid compounds of use in the tablets of the invention are preferably those which contain at least 18 carbon atoms in the carbon backbone. Lower chain fatty acids (those of 17 carbon atoms or less in the backbone) appear to show fewer useful therapeutic effects, but can be useful in applications like fish or animal feed.

Thus, preferred unsaturated fatty acid compounds are those of formula (I')

CH₃CH₂CH=CH-R-COOH (I')

wherein R is a C₁₃₊ alkylene group (e.g. C₁₃₋₂₅) optionally containing 1 or more double bonds, preferably non-conjugated;
or a derivative thereof.

Ideally, the R group is linear although it is within the scope of the invention for the backbone to carry alkyl side chains such as methyl or ethyl. The total number of carbon atoms in the chain is preferably 16 to 22. Moreover, R is preferably 13, 15, 17, 19 etc. i.e. the number of carbon atoms in the chain is preferably even. Whilst it will be appreciated that the omega 3 enriched tablets of the invention will, most likely, contain a variety of different omega-3 based compounds, highly preferred compounds of formula (I) are eicosapentaenoic acid (EPA) and docosahexaenoic acid (DHA) or derivatives thereof, e.g. triglyceride, phospholipid, sodium salt or polyamino alcohol salt thereof.

In a highly preferred embodiment, the fatty acid compounds comprise a mixture of DHA and EPA or derivatives thereof. The ratio of such compounds may be 30:70 to 70:30, preferably 40:60 to 60:40 EPA/DHA. The most mixtures of compounds are mixtures comprising at least EPA and DHA in the form of free acids, physiologically acceptable salts, ethyl esters, phospholipids and triglycerides.

The tablets of the invention may also contain omega-6 fatty acids. Preferred omega-6 fatty acids are those of formula (II):

CH₃CH₂CH₂CH₂CH₂CH=CH-R"-COOH (II)

wherein R" is a C₅₊ alkylene group (e.g. C₁₀₋₂₂) optionally containing 1 or more double bonds;
or derivatives thereof.

Ideally, the R" group is linear although it is within the scope of the invention for the backbone to carry alkyl side chains such as methyl or ethyl.

The number of carbon atoms in R" is preferably 10, 12, 14, 16 etc, i.e. the number of carbon atoms in the chain is preferably even. In a preferred embodiment the omega-6 fatty acid compound is ALA, gamma-linolenic acid (GLA) or conjugated linoleic acid (CLA), or a derivative thereof, e.g. a triglyceride, phospholipid, sodium salt or polyamino alcohol salt thereof.

Whilst it will be appreciated that the tablets of the invention will, most likely, contain a variety of different omega 3 and 6 based compounds, highly preferred compounds of formula (II) are C18, C20 and C22 compounds.

The weight ratio of omega-3 to omega-6 fatty acid compounds in the tablets of the invention may be of the order 1:1 to 1:3.

Preferably, the fatty acids of the invention will have at least 10 carbon atoms, e.g. at least 12 carbon atoms, such as at least 14 carbon atoms in the fatty acid portion of the molecule, i.e. a fatty acid must comprise at least 10 carbon atoms.

Ideally compounds of formula (I), (I') or (II) will be multiply unsaturated, e.g. contain 2 to 10 double bonds, especially 4 to 7 double bonds. Preferably double bonds are not conjugated either to each other or to the carbonyl functionality.

At least one, e.g. 2 or 3, preferably all double bonds are preferably in the cis configuration.

Crude oils contain a variety of fatty acids or derivatives thereof (e.g. esters thereof, in particular triglycerides) having differing carbon chain lengths and differing levels of unsaturation. Of course not all these fatty acids will be omega-3 unsaturated fatty acid compounds, some will be omega-6 unsaturated, some may be saturated oils. Tablets comprising a mixture of these fatty acid compounds are therefore covered

Whatever the nature of the fatty acid material, it is readily available from commercial sources. Pure DHA and EPA can be purchased and converted to an appropriate derivative using trivial chemistry.

The tablets of the invention may contain at least 10wt% fatty acid triglycerides (in total), e.g. at least 20 wt% or at least 25 wt% or at least 30 wt% or at least 40 wt% such as at least 50 wt% fatty acid triglycerides (in total).

The tablets of the invention preferably contain at least 100 mg, e.g. at least 125 mg, preferably at least 150 mg, such as at least 200 mg, e.g. at least 300 mg of fatty acid triglycerides (in total), preferably at least 400 mg, more preferably at least 500 mg, especially at least 600 mg.

In a preferred aspect of the present invention the tablets comprise omega-3 and/or omega-6 fatty acid triglycerides and cyclodextrin, especially in the form of a fatty acid compound cyclodextrin complex. Beta cyclodextrin is used in the invention. These are commercially available materials.

The term complex is used here to designate that fatty acid triglycerides are associated with the cyclodextrin through some form of intermolecular non-covalent bonds. These bonds include normally relative weak bonds like hydrophobic interactions. The fatty acid in the cyclodextrin complex is normally located within the core of the cyclodextrin molecule but could also be associated with other parts of the molecule. The most preferred size of the cyclodextrins is beta-cyclodextrin.

The cyclodextrins with different cavity size can optionally be substituted. The preferred substituent include alkyl groups, hydroxyalkyl groups, acyl groups For reviews on pharmaceutical acceptable cyclodextrin derivatives see: K.Uekama et al in J. Inclution Phenomena and Macrocyclic Chemistry (2006) 56:page 3-8,T.Loftsson et al. in Am. J.Drug Deliv. (2004)2:page 261-275 and J.Szejtli in J. Inclution Phenomena and Macrocyclic Chemistry (2005)52:1-11.

The most preferred cyclodextrin according to the present invention is unsubstituted beta-cyclodextrin and methyl or hydroxypropyl derivatives thereof.

The even most preferred cyclodextrins are beta-cyclodextrin and hydroxypropyl-cyclodextrin.

The weight ratio between fatty acid triglycerides (or compounds total) and cyclodextrin can vary over wide limits. The weight ratio is in the range of 1:5 to 5:1 (between fatty acid triglycerides (or compounds total) and cyclodextrin), preferably 1:2 to 2:1. In some embodiments the ratio between fatty acid triglycerides (or compounds total) and cyclodextrin is above 1.0:1, more preferably is above 1.2:1, even more preferably above 1.4:1, most preferably above 1.6:1. Thus, there may be more fatty acid triglycerides present than cyclodextrin. Surprisingly, this has been found still to result in a stable fatty acid compound composition which resists oxidation.

Without wishing to be limited by theory, it will be appreciated that as there is an excess of fatty acid triglycerides present relative to cyclodextrin some fatty acid may not be complexed and hence would be expected to oxidise readily. This is not observed however and it is thus highly surprising that complexes formed with an excess of fatty acid compound are suitable for use in the invention. The ratios above between fatty acid triglycerides and cyclodextrin therefore refer to the ratios between fatty acid triglycerides and cyclodextrin present. Whilst ideally in this embodiment, all the fatty acid compound present will be complexed, there is a possibility that some fatty acid material remains uncomplexed. This material is still counted in the ratios above or in the weight of a complex present.

The tablets of the invention comprise fatty acid triglycerides/cyclodextrin complex where the complex weight is more than 20% of the tablet weight, preferably more than 30%, such as greater than 40%. In some embodiments, the complex can form more than 70% of the tablet weight, preferably more than 80% of the tablet weight most preferably more than 90% of the tablet weight.

Complexes of fatty acid compounds with cyclodextrin are known in the art and hence techniques for their formulation are also known. A convenient method involves the use of water as a solvent for the cyclodextrin which can then be mixed with the fatty acids e.g. in the form of an ester. The complex forms and can be separated, e.g. by filtration and washed.

An alternative technique utilises organic solvent, e.g. an aqueous alcohol, in which both fatty acid compound and cyclodextrin can be refluxed. The formed complex can be collected by filtration after cooling.

Thus, the cyclodextrin complexes with fatty acid compounds can be prepared using state of the art techniques for preparation of cyclodextrin complexes. Typical methods include for example formation of the complex in water, in mixture of water and organic solvents or water-free organic solvents at ambient temperatures. Typical organic solvents include methanol, ethanol, isopropanol, acetone, DMSO, DMF and acetonitrile. The ratio between cyclodextrin and fatty acid triglycerides should preferably be low, typically below 1. The cyclodextrin complex with fatty acid triglycerides is isolated by filtration, evaporation or freeze drying.

The complex of fatty acid triglycerides with cyclodextrin is a solid, preferably a powder. In a highly preferred embodiment, the material is a crystalline solid. It should not be an oily material. It will be appreciated that sometimes to achieve a powder a solid may need to be ground. In a further preferred embodiment therefore the complex will be suitable for grinding to form a powder.

The formation of powder complexes of fatty acid triglycerides and cyclodextrin is new and these powders form intermediates in the preparation of the tablets of the invention.

The invention also provides a process for converting a fatty acid triglycerides oil into a solid comprising contacting in solution at least one fatty acid or derivative thereof with a cyclodextrin to form a complex thereof and drying to form a solid, preferably a powder.

Drying of the complex can be carried out by any known means. The material can be vacuum dried or simply left to dry in ambient air. It could be gently heated to encourage drying. Preferred drying methods include freeze drying and spray drying however. Spray drying techniques are disclosed in "Spray Drying Handbook", K. Masters, 5th edition, Longman Scientific Technical UK, 1991, the disclosure of which is referred to at least for its teaching of spray drying methods.

It will be appreciated that as there is more than one fatty acid triglyceride present, there can be more than one cyclodextrin complex formed. The present invention relates to tablets comprising cyclodextrin complexes with two or more fatty acid compounds. It is also within the scope of the invention for a mixture of cyclodextrins to be used, e.g. beta and gamma cyclodextrin or derivatives thereof.

Optionally as well as the use of cyclodextrins, the invention covers the use of calixarenes to form complexes with the fatty acid compounds. Calixarenes are macrocyclic compounds capable of assuming a basket (or "calix") shaped conformation. They are formed from p-hydrocarbyl phenols and formaldehyde and the term applies to a variety of compounds derived by substitution of the hydrocarbon cyclo {oligo[(1,3-phenylene) methylene]}.

A discussion of the use of calixarenes in complexation of amphilic molecules can be found in Nannelli et al, Molecular Crystals and Liquid Crystals (2001), 367 621-630.

Viewed from another aspect therefore the invention provides a pharmaceutical or nutraceutical tablet for oral administration as described herein further comprising more than 50% (weight) of at least one fatty acid or derivative thereof and at least one calixarene.

The ratios discussed above in relation to cyclodextrin complexation also apply mutatis mutandis to calixarene complexes.

In order to form tablets it is highly preferred if the fatty acid compound is in the form of a solid, especially a powder, especially a crystalline solid. This can be achieved through complex formation as described above or achieved by isolating a fatty acid compound in solid form.

The tablets of the invention may be produced by compression or compaction of a formulation containing the fatty acid triglycerides and/or complex thereof and certain excipients, typically selected to aid in the processing and to improve the properties of the tablet. The tablets of the invention may be coated or uncoated and can be made from powdered, crystalline materials. Tablets may be plain, film or sugar coated, bisected, embossed, layered, or sustained release. Any film coating preferably comprise of a physiologically acceptable water-soluble organic polymer They can be made in a variety of sizes, shapes and colours.

Excipients which may be present include diluents, binders, disintegrants, lubricants, glidants and in many cases, colorants. The excipients used are classified according to the function they perform. For example, a glidant may be used to improve the flow of powder blend in the hopper and into the tablet die.

Lubricants are typically added to prevent the tableting materials from sticking to punches, minimize friction during tablet compression, and allow for removal of the compressed tablet from the die. Such lubricants are commonly included in the final tablet mix in amounts usually less than 1% by weight. The most commonly used lubricants are magnesium stearate, stearic acid, hydrogenated oil, and sodium stearyl fumarate.

Tablets often contain diluents, such as lactose, which are added to increase the bulk weight of the blend resulting in a practical size for compression. This is often necessary where the dose of the drug is relatively small so the use of diluents is favoured in this invention where high doses of the fatty acid compounds are required. Typical diluents include for example dicalcium phosphate, calcium sulphate, lactose, cellulose, kaolin, mannitol, sodium chloride, dry starch and other sugars. The cellulose can preferably be microcrystalline cellulose (Avicel).

Binders are agents which impart cohesive qualities to the powdered material. Commonly used binders include starch, gelatin, sugars such as sucrose, glucose, dextrose, and lactose, natural and synthetic gums, carboxymethylcellulose, methylcellulose, polyvinylpyrrolidone, ethylcellulose and waxes..

Disintegrants are often included to ensure that the tablet has an acceptable rate of disintegration. Typical disintegrants include starch derivatives, crospovidone, croscaramelose and salts of carboxymethylcellulose. Some binders, such as starch and cellulose, are also excellent disintegrants.

Other desirable characteristics of excipients include high compressibility to allow strong tablets to be made at low compression forces, good flow properties that can improve the flow of other excipients in the formula and cohesiveness (to prevent tablet from crumbling during processing, shipping and handling). The skilled man knows the type of excipients appropriate for tablet formulation.

It is preferred if the total weight of excipients in a tablet of the invention is no more than 20 wt% of that tablet, preferably less than 15 wt% of the tablet, especially less than 10 wt% of the tablet.

The three processes for making compressed tablets are wet granulation, direct compression, and dry granulation (slugging or roller compaction). Whilst all three methods can be used to form the tablets of the invention, it is preferred if direct compression is employed.

Dry granulation consists of blending, slugging the ingredients, dry screening, lubrication, and compression. The wet granulation method is used to convert a powder mixture into granules having suitable flow and cohesive properties for tableting. The procedure consists of mixing the powders in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes: weighing, dry powder blending, wet granulating, drying, milling, blending lubrication and compression.

Direct compression is a relatively quick process where the powdered materials are compressed directly without changing the physical and chemical properties of the drug. The fatty acid triglycerides, direct compression excipients and any other auxiliary substances, such as a glidant and lubricant are blended, e.g. in a twin shell blender or similar low shear apparatus before being compressed into tablets.

The advantages of direct compression include uniformity of blend, few manufacturing steps involved, (i.e. the overall process involves weighing of powders, blending and compression, hence less cost), elimination of heat and moisture, prime particle dissociation, and physical stability.

However, direct compression is usually limited to those situations where the drug or active ingredient has a crystalline structure and physical characteristics required to form pharmaceutically acceptable tablets. Since the fatty acid triglycerides of the invention typically present as oils, the use of direct compression to form oral dosage forms of fatty acid compounds is not reported. Moreover, since excipients need to be added to a direct compression formulation to allow the compression process to take place manufacturers are often limited to using the direct compression method in formulations containing a low dose of the active ingredient per compressed tablet as otherwise tablet sizes become to large for swallowing.

A solid dosage form containing a high dose drug (i.e. where the drug itself comprises a substantial portion of the total compressed tablet weight) can only be directly compressed if the drug itself has sufficient physical characteristics (e.g. cohesiveness) for the ingredients to be directly compressed. Surprisingly, the inventors have found that fatty acid compounds and complexes of the invention possess the necessary physical characteristics. The fatty acid compounds and complexes of the invention have unexpectedly good flow and compression characteristics. The material, optionally mixed with excipients as described above, for example microcrystalline cellulose and magnesium stearate, is free-flowing and sufficiently cohesive to act as a binder..

It is surprisingly found therefore that fatty acid triglycerides which can be presented in solid form, especially as cyclodextrin complexes, especially complexes comprising high amounts of fatty acid triglycerides, can be tabletted without prior granulation (i.e. by direct compression). The most preferred method of production of tablets of the invention is therefore by direct compression.

The size of the tablets, according to the present invention can vary. The tablet diameter can vary from 6 mm to 20 mm, preferably 8 to 14 mm. The tablet weight can vary from 100 mg to 3 grams. The most preferred tablets have tablet weights between 200 mg and 2 grams with a diameter from 8 to 12 mm.

The tablets are for oral administration either by direct swallowing thereof or by any other known means, e.g. chewable tablets, dissolution or suspension of the tablet in a drinkable liquid and so on.

Whilst the tablets are primarily for use with human consumers, tablets might also be administered to animals, especially mammals, e.g. higher mammals.

In a further preferred aspect of the invention, tablets comprising fatty acid triglycerides can be formulated together with other active agents. Active agents which could be combined with the complexes of the invention include pharmaceuticals, nutraceuticals, vitamins, minerals and other health supplementing compounds. Combination with drugs is highly preferable.

The most preferred drugs to be formulated together with fatty acid triglycerides in tablets according to the present invention are drugs for treatment and/or prophylaxis of diseases in the cardiovascular system and in bone. Typical such drugs include ACE-inhibitors, like for example enalapril, angiotensin II receptor antagonists like losartan, beta-blockers like propranolol, plasma cholesterol reducing compounds like statins, typically simvastatin or atorvastatin, and bisphosphonates like for example alendronate. Other favourable drugs include glucosamine.

Highly preferred additional components in the compositions of the invention also include simvastatin, atorvastatin, glucosamine, vitamins and/or minerals in particular calcium.

Another preferred aspect of the present invention relates to tablets comprising fatty acid triglycerides of the invention together with these nutraceutical or pharmaceutical ingredients. Typical nutraceutical ingredients can be calcium, iron or other minerals, water-soluble vitamins like Vitamin B or Vitamin C, lipid-soluble vitamins like Vitamin A, D, K (e.g. K2) or E and ingredients present in the nature like for example herbs and extracts thereof.

In some embodiments it is also possible for an additional ingredient present in the compositions of the invention to be present as a cyclodextrin complex, especially where this is a vitamin, especially vitamin K2 and most especially MK-7.

This forms a still yet further aspect of the invention which therefore provides a complex formed between vitamin K2 and cyclodextrin, in particular a complex formed between MK-7 and cyclodextrin, especially a pharmaceutical or nutraceutical tablet for oral administration comprising such a complex.

These complexes can also be combined with fatty acid/cyclodextrin complexes to form especially preferred tablets of the invention.

The compositions of the invention may also contain folic acid and other well known over the counter health supplements such as echinacea.

The health benefits of the fatty acid compounds of the invention have been confirmed in many studies. Polyunsaturated fatty acids have been found to keep serum cholesterol levels low, stabilise irregular heartbeat, reduce blood pressure, improve autoimmune disease, improve depression disorders, treat psoriasis, treat rheumatoid arthritis, and to prevent colon cancer. They are generally applied in cardiovascular disorders and for the treatment of bone disorders. The tablets of the invention are of particular interest in the treatment or prevention of hypertriglyceridemia and cardiac infection. Hypertriglyceridemia is a medical condition characterized by increased plasma concentration of triglycerides.

The invention is further illustrated by the following non-limiting examples:

### Example 1 (outside of the scope of claim 1)

### EPA and DHA-ethyl ester complex with beta-cyclodextrin. Ratio 1:1 fatty acid derivative and cyclodextrin

Beta-cyclodextrin (5.0 grams) was dissolved in water (200 ml). The solution was cooled to room temperature. A mixture of EPA- and DHA-ethyl ester (Omacor®) (5.0 grams) was added. The mixture was stirred under an atmosphere of argon in the dark for 3 days. The product was isolated by centrifugation and dried. The product was a white powder.

### Example 2. (outside of the scope of claim 1)

### EPA and DHA-ethyl ester complex with beta-cyclodextrin. Ratio 5:2 fatty acid derivative and cyclodextrin .

The product was prepared as in Example 1 using a ratio of 5:2 fatty acid derivative and cyclodextrin . The product was isolated by freeze drying.. The product was a white powder

### Example 3 (outside of the scope of claim 1)

### EPA and DHA- ethyl ester complex with beta-cyclodextrin. Ratio 5: 1.5 Fatty acid derivative and cyclodextrin.

The product was prepared as Example 1 using a ratio of 5:1.5 fatty acid derivative and cyclodextrin. The product was isolated by filtration and dried. The product was a white powder.

### Example 4 (outside of the scope of claim 1)

### EPA- and DHA- ethyl ester complex with beta-cyclodextrin. Ratio 5:1.5 Fatty acid derivative and cyclodextrin.

The product was prepared as Example 3 using methanol instead of water. The product was a white solid.

### Example 5 (outside of the scope of claim 1)

### Preparation of tablet comprising EPA- and DHA ethyl ester complex with betacyclodextrin by direct compression of product.

A tablet (6 mm diameter, 300 mg tablet weight) was prepared by direct compression of the complex of example 1 or 2. Piston pressure was 0.5 tons. The tablet had good mechanical properties.

### Example 6 (outside of the scope of claim 1)

### Tablet disintegration

The disintegration time for tablet prepared in Example 5 was determined at 37 °C according to Ph. Eur procedure. The disintegration time was 40 minutes.

### Example 7 (outside of the scope of claim 1)

| | One tablet | 100 000 tablets |
|---|---|---|
| EPA/DHA ethyl ester beta-cyclodextrin complex(Example 2) | 650 mg | 650000g |
| Microcrystalline cellulose (Avicel PH-101) | 60 mg | 60000g |
| Stearic acid | 10 mg | 1000g |
| Colloidal silica (Cab-O-Sil) | 2 mg | 200g |

All ingredients are blended. Tablets are compressed using a Killian rotary tablet machine with 10 mm standard concave punch. 10 tablets weigh 7.22 g.

### Example 8 (outside of the scope of claim 1)

### EPA-and DHA ethyl ester complex with beta-cyclodextrin. Ratio 5:8 fatty acid derivative and cyclodextrin.

A complex between EPA- and DHA ethyl esters and beta-cyclodextrin was prepared by refluxing EPA- and DHA-ethyl esters (500 mg) from Omacor® (Pfizer, Norway) and beta-cyclodextrin (800 mg) for 1 hour in methanol (100 ml). The methanol was evaporated and the title compound isolated.

### Example 9 (outside of the scope of claim 1)

### Preparation of tablet comprising simvastatin and EPA- and DHA- ethyl ester complex with beta-cyclodextrin.

Simvastatin tablets 40 mg (from Ratiopharm GmbH, Ulm, Germany) were pulverized using a mortar and pestle. EPA- and DHA ethyl ester beta-cyclodextrin complex was added. The powder was mixed in the mortar and tablets were prepared. A mixture of tablet powder (600 mg) and EPA-and DHA ethyl ester beta-cyclodextrin complex (200 mg) were pressed to tablets comprising 59 mg simvastatin and 125 mg EPA- and DHA ethyl ester. Tablet diameter: 13 mm.

### Example 10. (outside of the scope of claim 1)

### Tablets comprising simvastatin and EPA and DHA ethyl ester complex with beta-cyclodextrin.

Tablets were prepared as in Example 9 based on tablet powder (400 mg) and EPA-and DHA ethyl ester beta cyclodextrin complex (400 mg). Each tablet contained 39 mg simvastatin and 250 mg EPA- and DHA ethyl ester. Tablet diameter: 13 mm.

### Example 11.

### Complex between oil comprising omega-3 fatty acid (Denomega®) and beta-cyclodextrin

Beta-cyclodextrin (20 gram) and water (5 ml) were mixed in a mortar for two minutes. Denomega® (Borregard, Norway) (10 gram) was added and vigorously mixed in the mortar with the pestle for 30 minutes. The product was dried in vacuum at 50°C overnight.

### Example 12.

### Tablets comprising simvastatin and Denomega® betacyclodextrin complex.

Tablets were prepared as in Example 9 based on tablet powder (250 mg) and Denomega® betacyclodextrin complex (250 mg). Each tablet contained 24 mg simvastatin and 83 mg Denomega® oil. Tablet diameter 13 mm.

### Example 13.

### Tablets comprising simvastatin and Denomega® beta-cyclodextrin complex.

Tablets were prepared as in Example 12 from 400 mg simvastatin tablet powder and 400 mg Denomega® beta-cyclodextrin complex. Each tablet contained 39 mg simvastatin and 133 mg Denomega® oil. Tablet diameter 13 mm.

### Example 14

### Tablets comprising simvastatin and Denomega® beta-cyclodextrin complex.

Tablets were prepared as in Example 12 from 700 mg simvastatin tablet powder and 100 mg Denomega® beta-cyclodextrin complex. Each tablet contained 68 mg simvastatin and 33 mg Denomega® oil. Tablet diameter 13 mm.

### Example 15.

### Complex between omega-3 fatty acid enriched cod liver oil and beta-cyclodextrin. Ratio 1 to 1 oil and beta-cyclodextrin

Beta-cyclodextrin (50 gram) and water (50 ml) were mixed in a mortar for five minutes. Omega-3 fatty acid enriched cod liver oil (Mollers Omega-3 , Moller, Norway) (50 gram) was added and vigorously mixed in the mortar with the pestle for 30 minutes. The product was dried in vacuum at 50 degrees centigrade overnight.

### Example 16

### Tablets comprising simvastatin and omega-3 fatty acid enriched cod liver oil beta-cyclodextrin complex.

Tablets were prepared as in Example 14 from 250 mg simvastatin tablet powder and 250 mg omega-3 fatty acid enriched cod liver oil beta-cyclodextrin complex (from Example 15.). Each tablet contained 24 mg simvastatin and 125 mg omega-3 fatty acid enriched oil. Tablet diameter 13 mm.

### Example 17

### Complex between omega-3 fatty acid enriched cod liver oil and beta-cyclodextrin. Ratio 1 to 4 oil to beta-cyclodextrin

Beta-cyclodextrin (2 gram) and omega-3 fatty acid enriched cod liver oil (500 mg) are added to 2-propanol (100 ml). The mixture was refluxed for 1 hour. The mixture was evaporated and dried at vacuum overnight. The title compound was isolated as a white powder.

### Example 18

### Tablets comprising simvastatin and omega-3 fatty acid enriched cod liver oil beta-cyclodextrin complex.

Tablets were prepared as in Example 16 from 600 mg simvastatin tablet powder and 200 mg omega-3 fatty acid enriched cod liver oil beta-cyclodextrin complex (from Example 17.). Each tablet contained 58 mg simvastatin and 40 mg omega-3 fatty acid enriched oil. Tablet diameter 13 mm.

### Example 19

### Tablets comprising simvastatin and omega-3 fatty acid enriched cod liver oil beta-cyclodextrin complex.

Tablets were prepared as in Example 18 from 400 mg simvastatin tablet powder and 400 mg omega-3 fatty acid enriched cod liver oil beta-cyclodextrin complex (from Example 17.). Each tablet contained 39 mg simvastatin and 80 mg omega-3 fatty acid enriched oil. Tablet diameter 13 mm.

### Examples 20-22

### Multivitamin tablets comprising omega-3 fatty acid enriched cod liver oil beta-cyclodextrin complex.

Multivitamin tablets (Nycoplus® Multi (Nycomed Pharma AS, Asker, Norway) are tablets comprising 11 vitamins and 8 minerals. One tablet covers the body's need for important vitamins and minerals. The tablets were pulverized in a mortar with a pestle and mixed with omega-3 fatty acid enriched cod liver oil beta-cyclodextrin complex (from Example 17). The mixture was compressed to tablets comprising various amounts of vitamins, minerals and omega-3 fatty acid enriched cod liver oil. All tablets: 13 mm diameter
Each tablet contained:
Example 20 : 13% more vitamins and minerals than Nycoplus®Multi tablets plus 20 mg cod liver oil .
Example 21: 81% of the vitamins and minerals in Nycoplus®Multi tablets plus 60 mg cod liver oil.
Example 22: 97% of the vitamins and minerals in Nycoplus® Multi tablets plus 80 mg cod liver oil.

### Example 23

### Complex between omega-3 fatty acid enriched cod liver oil and beta-cyclodextrin. Ratio 1 to 3 oil to beta-cyclodextrin

Beta-cyclodextrin (1.5 gram) and omega-3 fatty acid enriched cod liver oil(500 mg) are added to 2-propanol (100 ml). The mixture was refluxed for 1 hour. The mixture was evaporated and dried at vacuum over night. The title compound was isolated as a white powder.

### Example 24

### Multivitamin tablets comprising omega-3 enriched fatty acid cod liver oil

The tablets were prepared from Multivitamin tablets (Nycoplus® Multi (Nycomed Pharma AS, Asker, Norway) and omega-3 fatty acid enriched cod liver oil beta-cyclodextrin complex (Example 23) as described in Examples 20-22. Each tablet contained: 21% more vitamins and minerals than Nycoplus®Multi tablets plus 12.5 mg cod liver oil. Tablet diameter: 13 mm.

### Examples 25-27

### Vitamin C tablets comprising omega-3 fatty acid enriched cod liver oil beta-cyclodextrin complex.

Vitamin C tablets (Nycoplus® C-vitamin (Nycomed Pharma AS, Asker, Norway) are tablets comprising 250 mg vitamin C. The tablets were pulverized in a mortar with a pestle and mixed with omega-3 fatty acid enriched cod liver oil beta-cyclodextrin complex (from Example 23). The mixture was compressed to tablets comprising various amounts of vitamins, minerals and omega-3 fatty acid enriched cod liver oil. All tablet diameters: 13 mm
Each tablet contained:
Example 25 : 542 mg vitamin C plus 25 mg cod liver oil .
Example 26: 456 mg vitamin C plus 50 mg cod liver oil.
Example 27: 380 mg vitamin C plus 75 mg cod liver oil.

### Example 28-30

### Tablets comprising calcium carbonate and omega-3 fatty acid enriched cod liver oil beta-cyclodextrin complex.

Weifa Complete® (Weifa, Oslo, Norway) are calcium tablets comprising 250 mg calcium in the form of calcium carbonate, 50 microgram Vitamin K1 and 2.5 microgram vitamin D3. The tablets were pulverized in a mortar with a pestle and mixed with omega-3 fatty acid enriched cod liver oil beta-cyclodextrin complex (from Example 23). The mixture was compressed to tablets comprising various amounts of vitamins, minerals and omega-3 fatty acid enriched cod liver oil. All tablets: 13 mm diameter
Each tablet contained:
Example 28 : 90% of the vitamins and minerals in Weifa Complete® tablets plus 25 mg cod liver oil .
Example 29: 77% of the vitamins and minerals in Weifa Complete® tablets plus 50 mg cod liver oil.
Example 30: 64% of the vitamins and minerals in Weifa Complete® plus 75 mg cod liver oil.

### Example 31

### Complex between omega-3 fatty acid enriched cod liver oil and beta-cyclodextrin. Ratio 1:5 oil to cyclodextrin

Beta-cyclodextrin (250 gram) and water (250 ml) were mixed in a mortar for 30 minutes using a pestle. Omega-3 fatty acid enriched cod liver oil (62.5 gram) (Møllers Omega-3 tran, Moller, Norway) was added and was mixed in the mortar for 2 hours. The product was dried in vacuum. The title compound was isolated as a white powder.

### Example 32.

### Tablets comprising calcium carbonate and omega-3 fatty acid enriched cod liver oil beta-cyclodextrin complex.

Tablets were prepared from Weifa Complete® (Weifa, Oslo, Norway) powder (300 mg) and omega-3 fatty acid enriched cod liver oil (Example 31) (500 mg) as described in Examples 20-30. Each tablet contained 39 % of the mineral and vitamins in Weifa Complete® plus 100 mg omega-3 fatty acid enriched cod liver oil.

### Example 33 and 34. Tablets comprising folic acid and omega-3 fatty acid enriched cod liver oil.

Nycoplus® Folsyre (Nycomed AS, Asker, Norway) are tablets containing 0.4 mg folic acid per tablet. The tablets were pulverized in a mortar with a pestle and mixed with omega-3 fatty acid enriched cod liver oil beta-cyclodextrin complex (from Example 23). The mixture was compressed to tablets comprising various amounts of folic acid and omega-3 fatty acid enriched cod liver oil. All tablets: 13 mm diameter Each tablet contained:
Example 33: 2.9 mg folic acid and 25 mg omega-3 fatty acid enriched cod liver oil.
Example 34: 2.5 mg folic acid and 50 mg omega-3 fatty acid enriched cod liver oil.

### Example 35. Tablets comprising B vitamins and omega-3 fatty acid enriched cod liver oil.

Nycoplus® B-total (Nycomed As, Asker, Norway) are tablets containing various B vitamins. The tablets were pulverized in a mortar with a pestle and mixed with omega-3 fatty acid enriched cod liver oil beta-cyclodextrin complex (from Example 23). The mixture was compressed to tablets. Tablet diameter: 13 mm, tablet weight 800 mg. Each tablet contained: 4 times more B vitamins than Nycoplus® B-total plus 50 mg omega-3 fatty acid enriched cod liver oil.

### Example 36.

### Tablets comprising calcium, vitamin D and omega-3 fatty acid oil

Weifa Kalsium Vitamin D (Weifa AS, Oslo, Norway) are tablets comprising calcium in form of calcium carbonate and vitamin D3. Each tablet contained 250 mg calcium and 2.5 microgram vitamin D3. The tablets were pulverized in a mortar with a pestle and mixed with Denomega® beta-cyclodextrin complex (from Example 11). The mixture was compressed to tablets. Tablet diameter: 13 mm, tablet weight 800 mg. Each tablet contained 78% of the amount of calcium and Vitamin D3 present in Weifa Kalsium Vitamin D3 plus 67 mg Denomega® oil.

### Example 37

### Tablets comprising calcium and omega-3 fatty acid oil

Weifa Kalsium (Weifa AS, Oslo, Norway) are tablets comprising calcium in form of calcium carbonate. Each tablet contained 250 mg calcium. The tablets were pulverized in a portar with a pistle and mixed with Denomega® beta-cyclodextrin complex (from Example 11). The mixture was compressed to tablets.Tablet diameter: 13 mm, tablet weight 800 mg. Each tablet contained 78% of the amount of calcium present in Weifa Kalsium plus 67 mg Denomega® oil.

### Example 38. (outside of the scope of claim 1)

### Complex of EPA-/DHA- ethyl esters with betacyclodextrin (1:2)

EPA- and DHA ethyl ester (2.5 gram) (Omacor®, Pfizer, Norway) and beta-cyclodextrin (5 gram) and acetone (200 ml) were stirred for 5 hours at 40 degrees centigrade. The solution was evaporated and the title compound isolated after drying.

### Example 39. (outside of the scope of claim 1)

### Tablets comprising calcium and EPA-/DHA ethyl esters

Tablets were prepared as described in Example 37 from 600 mg Weifa Kalsium powder and 200 mg EPA-/DHA beta cyclodextrin (from Example 38) The tablets contained 192 mg calcium and 66 mg EPA-/DHA-ethyl ester.

### Example 40a

### Complex of omega-3 (Nycoplus omega-3) beta-cyclodextrin .Ratio 1 to 1.

Nycoplus®Omega-3 +ACDE (Nycomed, Asker, Norway) are capsules comprising omega-3 oil and several vitamins. The oil from these capsules (5.0 gram), beta-cyclodextrin (5.0 gram) and methanol (100 ml) were refluxed for one hour and evaporated. The title compound isolated.

### Example 40b. Complex of omega-3 (Nycoplus omega-3) betacyclodextrin. Ratio: 1 to 1.

Beta-cyclodextrin (5.0 gram) and water (2.5 ml) were added into a mortar and mixed with a pestle for 5 minutes. Nycoplus®Omega-3+ ACDE (5.0 grams) were added and mixed for 30 minutes. The powder was dried in vacuum.

### Example 41.

### Tablets comprising calcium, vitamin A, vitamin C, vitamin D, vitamin E, vitamin K and omega-3 triglycerides

Nycoplus® calcium + vitamin K and vitamin D (Nycomed AS, Asker, Norway) are tablets comprising calcium carbonate, vitamin K and vitamin D. These tablets (750 mg) were crushed in a mortar and mixed with Nycoplus®Omega3 + ACDE beta-cyclodextrin powder (from Example 40b). A tablet was prepared. Tablet diameter 13 mm, tablet weight 800 mg.

### Example 42.

### Tablets comprising calcium, vitamin A, vitamin C, vitamin D and vitamin E

Tablets were prepared as described in Example 37 from Weifa Kalsium with vitamin D powder (600 mg) and Nycoplus®Omega-3+ACDE beta-cyclodextrin (from Example 40B) (200 mg) powder. Tablet diameter 13 mm, tablet weight 800 mg.

### Example 43

### Tablets comprising calcium, vitamin A, vitamin C, vitamin D, vitamin E and omega-3 fatty acid oil

Tablets were prepared as described in Example 42. Weifa Kalsium and not Weifa Kalsium with vitamin D was used.

### Example 44.

### Tablets comprising vitamin D and omega-3 fatty acid

Nycoplus® D-vitamin (Nycomed,Asker,Norway) are tablets containing 10 microgram vitamin D in each tablet. The tablets were pulverized in a mortar with a pestle (600 mg powder) and mixed with Nycoplus®omega-3 +ACDE beta-cyclodextrin complex (from Example 40B) (100 mg). The mixture was compressed to tablets. Tablet diameter: 13 mm, tablet weight 700 mg.

### Example 45a.

### Complex between omega-3 oil (Weifa Complete® Godt for leddene) and beta-cyclodextrin. Ratio 1 to 1.

Weifa Complete® Godt for leddene (Weifa AS, Oslo, Norway) are capsules with omega-3 fatty acids as main component. In addition, this product contains some ginger. Weifa Complete®Godt for leddene oil (5.0 gram), beta-cyclodextrin (5.0 gram) and methanol (100 ml) were refluxed for 1 hour. The mixture was evaporated. The title compound was dried in vacuum over night.

### Example 45b.

### Complex between omega-3 oil(Weifa complete®Godt for leddene) and beta-cyclodextrin. Ratio 1:1

Beta-cyclodextrin (5.0 gram) and water (2.5 gram) were added into a mortar and mixed for 5 minutes using a pestle. Weifa Complete®Godt for leddene oil (5.0 gram) was added and mixed for 30 minutes using the same pestle. The product was dried in vacuum.

### Example 46.

### Tablets comprising calcium, vitamin D, ginger and omega-3.

Tablets were prepared as described in Example 37 from Weifa Kalsium with vitamin D (600 mg powder) and Weifa Complete®Godt for leddene beta-cyclodextrin (from Example 45B) (200 mg) powder. Tablet diameter 13 mm, tablet weight 800 mg.

### Example 47.

### Tablets comprising calcium, ginger and omega-3.

Tablets were prepared as described in Example 37 from Weifa Kalsium (600 mg powder) and Weifa Complete®Godt for leddene beta-cyclodextrin (from Example 45b) (200 mg) powder. Tablet diameter 13 mm, tablet weight 800 mg.

### Example 48.

### Preparation of omega-3 betacyclodextrin complex in industrial scale

Beta-cyclodextrin (250 gram) and water (250 ml) were mixed in a mortar for 30 minutes. Omega-3 fatty acid enriched cod liver oil (62.5 gram) was added and mixed for 2 hours using a pestle. The product was dried in vacuum at 50°C. The title compound was isolated as a white powder.

### Example 49.

### Preparation of tablets comprising omega-3 fatty acid enriched cod liver oil beta-cyclodextrin complex in industrial scale

Omega-3 fatty acid enriched cod liver oil betacyclodextrin complex (from Example 48) 260 gram, Microcrystalline cellulose MCC PH 102 Emcocel 1400 gram, Magnesium stearate 33 gram. The omega-3 component was carefully sieved to remove aggregates. The above components were thoroughly mixed and used for direct compression of tablets. The tablets were prepared on a Manesty B3B tablet press. The average tablet weight was 345 mg and the tablet diameter was 10 mm. Approximately 5000 tablets were prepared. The tablets were of good technical quality. The mechanical strengh of the tablets were acceptable. Disintegration time was determined according to standard pharmacopoeian procedure was shorter than 1 minute.

### Example 50

### Tablets comprising atorvastatin and omega-3

Tablets prepared in Example 49 were pulverized in a mortar with a pestle and mixed with amorphous calcium atorvastatin. The mixture was compressed to tablets. Tablet diameter was 13 mm and tablet weight 680 mg. Each tablet contained 40 mg calcium atorvastatin

### Examples 51-56. (outside of the scope of claim 1)

### Vitamin K2 (MK7) cyclodextrin complexes

VitaminK2 (Menaquinone-7,MK7, CAS NO 2124-57-4) was bought from Wako Pure Chemical Industries,Ltd, Osaka, Japan). Cyclodextrin and water were mixed in a mortar for 5 minutes, menaquinone-7 was added and mixed for 15 minutes. The products were dried. Components used for preparation of the various menaquinone-7 cyclodextrin complexes are:
Example 51: MK-7 (1 mg), beta-cyclodextrin 10 mg, water 50 mg.
Example 52: MK-7 (1 mg), beta-cyclodextrin 50 mg, water 100 mg
Example 53: MK7 (1 mg), beta-cyclodextrin 100 mg, water 250 mg
Example 54: MK7 (1 mg), beta-cyclodextrin 1000 mg, water 500 mg
Example 55: MK7 (1 mg), gamma-cyclodextrin 100 mg,
Example 56: MK7(1mg), 2-hydroxypropyl-beta-cyclodextrin 1000 mg

The compositions of the final MK7 cyclodextrin complexes were as above, water content was not determined.

### Example 57

### Tablets comprising vitamin K2 (menaquinone-7) cyclodextrin complex and omega-3

Tablets prepared in Example 49 were pulverized in a mortar with a pestle and mixed with MK7 cyclodextrin complex (100 mg) (from Example 54). The mixture was compressed to tablets. Tablet diameter was 13 mm and tablet weight 740 mg. Each tablet contained 0.1 mg MK7.

### Example 58

### Tablets comprising vitamin K2 (menaquinone 7) cyclodextrin complex and omega-3

Tablets prepared in Example 49 were pulverized in a mortar with a pestle and mixed with MK7 cyclodextrin complex (10 mg) (from Example 54). The mixture was compressed to tablets. Tablet diameter was 13 mm and tablet weight 650 mg. Each tablet contained 10 microgram MK7.

### Example 59. Tablets comprising calcium, vitamin A, vitamin D and Vitamin K2 (MK7) in form of betacyclodextrin complex.

Tablets were prepared as described in Example 37 from Nycoplus® calcium + vitamin K or vitamin D and MK7 beta-cyclodextrin (from Example 54)(100 mg) powder. Tablet diameter 13 mm, tablet weight 900 mg. Each tablet contained 0.1 mg MK7 in form of beta-cyclodextrin complex.

### Example 60.

### Tablets comprising alendronate and omega-3

Tablets prepared in Example 49 were pulverized (640 mg) in a mortar with a pestle and mixed with one pulverized Fosamax® tablets (10 mg alendronate). The mixture was compressed to tablets. Tablet diameter was 13 mm. Each tablet contained 10 mg alendronate.

### Example 61.

### Tablets comprising alendronate (10 mg) and omega-3 fatty acid (One tablet per day)

Tablets prepared in Example 49 were pulverized in a mortar with a pestle and mixed with one pulverized Fosamax® tablets (10 mg alendronate). The mixture was compressed to tablets. Tablet diameter was 13 mm.

Each tablet contains:
Example 61: Pulverized omega-3 tablets 960 mg, aldendronate 10 mg, total tablet weight: 1158 mg

### Example 62-63.

### Tablets comprising alendronate (10 mg) and omega-3 fatty acid (one tablet per week)

Tablets prepared in Example 49 were pulverized in a mortar with a pestle and mixed with one pulverized Fosamax® tablets (70 mg alendronate). The mixture was compressed to tablets. Tablet diameter was 13 mm.

Each tablet contains:
Example 62: Pulverized omega-3 tablets 640 mg, alendronate 70 mg, total tablet weight: 983 mg
Example 63: Pulverized omega-3 tablets 960 mg, aldendronate 70 mg, total tablet weight: 1303 mg
Example 64. Preparation of omega-3 betacyclodextrin complex (ratio 25:20 oil to beta-cyclodextrin)

Beta-cyclodextrin (20 gram) and water (20 ml) were mixed in a mortar for 2 minutes. Omega-3 fatty acid enriched cod liver oil (25 gram) was added and mixed for 30 minutes using a pestle. The product was dried in vacuum at 50°C. The title compound was isolated.

### Example 65.

Preparation of tablet comprising more than 500 mg omega-3 fatty acid enriched cod liver oil beta-cyclodextrin complexes

| | |
|---|---|
| Omega-3 fatty acid enriched cod liver oil betacyclodextrin complex (from Example 48) (ratio 1:4 oil to beta-cyclodextrin) | 53 mg |
| Omega-3 fatty acid enriched cod liver oil beta-cyclodextrin complex | |
| (from Example 64) (ratio 25:20 oil to beta-cyclodextrin) | 1237 mg |
| Microcrystalline cellulose MCC PH 102 Emcocel | 285 mg |
| Magnesium stearate | 6.7 mg |

A tablet was prepared by direct compression (10 tons)
The tablet contained 700 mg omega-3.

### Example 66.

### Preparation of tablet comprising more than 500mg omega-3 fatty acid compounds in the form of EPA-/DHA ethyl ester beta-cyclodextrin complex and triglyceride beta-cyclodextrin complex .

| | |
|---|---|
| Omega-3 fatty acid enriched cod liver oil betacyclodextrin complex (from Example 48) (ratio 1:4 oil to beta-cyclodextrin) | 53 mg |
| EPA-/DHA ethyl ester beta-cyclodextrin complex (from Example 38) | 1500 mg |
| Microcrystalline cellulose MCC PH 102 Emcocel | 285 mg |
| Magnesium stearate | 6.7 mg |

A tablet was prepared by direct compression (10 tons)
The tablet contained 510 mg omega-3 fatty acid compounds.

### Example 67. Preparation of tablet comprising cod liver oil beta-cyclodextrin complex and glucosamin.

Glucosamin Mezina 400mg (Mexina UK Ldt, London, UK) are tablets comprising glucosamin sulphate potassium chloride. Each tablet contains 400 mg glucosamin. One tablet was crushed in a mortar and mixed with:

| | |
|---|---|
| Omega-3 fatty acid enriched cod liver oil betacyclodextrin complex (from Example 48) (ratio 1:4 oil to beta-cyclodextrin) | 53 mg |
| Microcrystalline cellulose MCC PH 102 Emcocel | 285 mg |
| Magnesium stearate | 6.7 mg |

A tablet was prepared by direct compression
The tablet contained 400 mg glucosamin and 53 mg omega-3 fatty acid enriched omega-3 fatty acid oil beta-cyclodextrin complex.

### Example 68. (outside of the scope of claim 1)

### Complex between EPA and beta-cyclodextrin. Ratio 1 to 1 EPA and beta-cyclodextrin.

Beta-cyclodextrin (3 gram) and water (1 ml) were mixed in a mortar for five minutes. EPA (3 gram) was added and vigorously mixed in the mortar with the pistle for 10 minutes. After drying the material was a powder.

### Example 69 Tablets comprising simvastatin and EPA

Sinvastatin powder (500 mg) from crushed simvastatin tablets (ratiopharm,Ulm,Germany) comprising 48.8 mg simvastatin was mixed with:

| | |
|---|---|
| Omega-3 fatty acid enriched cod liver oil betacyclodextrin complex (from Example 48) (ratio 1:4 oil to beta-cyclodextrin | 53 mg |
| Microcrystalline cellulose MCC PH 102 Emcocel | 285 mg |
| Magnesium stearate | 6.7 mg |

A tablet was prepared by direct compression.

## Claims

1. A pharmaceutical or nutraceutical tablet for oral administration comprising at least 20 wt% of solid complex formed between at least two fatty acid triglycerides and at least one cyclodextrin, wherein the content of said at least two fatty acid triglycerides in said complex is 10 wt% or more, wherein the weight ratio of fatty acid compounds (total) to cyclodextrin is 1:5 to 5:1 and wherein the cyclodextrin is a beta-cyclodextrin.

2. A pharmaceutical or nutraceutical tablet for oral administration as claimed in any preceding claim comprising more than 170 mg of at least two fatty triglycerides and at least one cyclodextrin.

3. A pharmaceutical or nutraceutical tablet as claimed in claim 1 to 2 comprising DHA and/or EPA triglycerides.

4. A pharmaceutical or nutraceutical tablet as claimed in any of the preceding claims where said tablet comprises an additional active agent such as a pharmaceutical, nutraceutical, vitamin, mineral or other health supplementing compound.

5. A pharmaceutical or nutraceutical tablet as claimed in claim 4 wherein the active agent is selected from a calcium salt, simvastatin, atorvastatin, glucosamine, at least one vitamin, and folic acid.

6. A pharmaceutical or nutraceutical tablet as claimed in any preceding claim wherein said at least one fatty acid comprises an omega-3 fatty acid.

7. A pharmaceutical or nutraceutical tablet as claimed in any preceding claim wherein said at least one fatty acid comprises an omega-3 fatty acid and at least one omega-6 fatty acid.

8. A pharmaceutical or nutraceutical tablet according to any preceding claim prepared by direct compression.

9. A pharmaceutical or nutraceutical tablet as claimed in claim 5 comprising calcium carbonate.

10. A method for preparation of a pharmaceutical or nutraceutical tablet described in any of claims 1 to 9 comprising directly compressing at least 20 wt% of solid complex formed between at least two fatty acid triglycerides and at least one cyclodextrin, wherein the content of said at least two fatty acid triglycerides in said complex is 10 wt% or more, wherein the weight ratio of fatty acid compounds (total) to cyclodextrin is 1:5 to 5:1. and wherein the cyclodextrin is a beta-cyclodextrin.

11. Pharmaceutical tablet as claimed in claim 1 to 9 for use in the treatment and prophylactic treatment of diseases of the cardiovascular system and bone.

## Patentansprüche

1. Pharmazeutische oder nutrazeutische Tablette zur oralen Verabreichung, umfassend mindestens 20 Gew.-% eines zwischen mindestens zwei Fettsäuretriglyceriden und mindestens einem Cyclodextrin gebildeten Feststoffkomplexes, wobei der Gehalt an den mindestens zwei Fettsäuretriglyceriden in dem Komplex 10 Gew.-% oder mehr beträgt, wobei das Gewichtsverhältnis von Fettsäureverbindungen (gesamt) zu Cyclodextrin 1:5 bis 5:1 beträgt und wobei das Cyclodextrin ein Beta-Cyclodextrin ist.

2. Pharmazeutische oder nutrazeutische Tablette zur oralen Verabreichung nach einem vorstehenden Anspruch, umfassend mehr als 170 mg von mindestens zwei Fettsäuretriglyceriden und mindestens einem Cyclodextrin.

3. Pharmazeutische oder nutrazeutische Tablette nach Anspruch 1 bis 2, umfassend DHA- und/oder EPA-Triglyceride.

4. Pharmazeutische oder nutrazeutische Tablette nach einem der vorstehenden Ansprüche, wobei die Tablette einen zusätzlichen Wirkstoff wie zum Beispiel eine pharmazeutische, nutrazeutische, Vitamin-, Mineral- oder andere Gesundheitszusatzbindung umfasst.

5. Pharmazeutische oder nutrazeutische Tablette nach Anspruch 4, wobei der Wirkstoff aus einem Calciumsalz, Simvastatin, Atorvastatin, Glucosamin, mindestens einem Vitamin und Folsäure ausgewählt ist.

6. Pharmazeutische oder nutrazeutische Tablette nach einem vorstehenden Anspruch, wobei die mindestens eine Fettsäure eine Omega-3-Fettsäure umfasst.

7. Pharmazeutische oder nutrazeutische Tablette nach einem vorstehenden Anspruch, wobei die mindestens eine Fettsäure eine Omega-3-Fettsäure und mindestens eine Omega-6-Fettsäure umfasst.

8. Pharmazeutische oder nutrazeutische Tablette nach einem vorstehenden Anspruch, hergestellt durch direkte Kompression.

9. Pharmazeutische oder nutrazeutische Tablette nach Anspruch 5, umfassend Calciumcarbonat.

10. Verfahren zur Herstellung einer in einem der Ansprüche 1 bis 9 beschriebenen pharmazeutischen oder nutrazeutischen Tablette, umfassend das direkte Komprimieren von mindestens 20 Gew.-% des zwischen mindestens zwei Fettsäuretriglyceriden und mindestens einem Cyclodextrin gebildeten Feststoffkomplexes, wobei der Gehalt an den mindestens zwei Fettsäuretriglyceriden in dem Komplex 10 Gew.-% oder mehr beträgt, wobei das Gewichtsverhältnis von Fettsäureverbindungen (gesamt) zu Cyclodextrin 1:5 bis 5:1 beträgt und wobei das Cyclodextrin ein Beta-Cyclodextrin ist.

11. Pharmazeutische oder nutrazeutische Tablette nach Anspruch 1 bis 9 zur Verwendung bei der Behandlung und prophylaktischen Behandlung von Krankheiten des Herzkreislaufsystems und der Knochen.

## Revendications

1. Comprimé pharmaceutique ou nutraceutique pour administration orale comprenant au moins 20% en poids de complexe solide formé entre au moins deux triglycérides d'acide gras et au moins une cyclodextrine, dans lequel la teneur desdits au moins deux triglycérides d'acide gras dans ledit complexe est de 10 % en poids ou plus, dans lequel le rapport pondéral de composés d'acide gras (total) sur la cyclodextrine est de 1:5 à 5:1 et dans lequel la cyclodextrine est une bêta-cyclodextrine.

2. Comprimé pharmaceutique ou nutraceutique pour administration orale selon l'une quelconque des revendications précédentes comprenant plus de 170 mg d'au moins deux triglycérides gras et au moins une cyclodextrine.

3. Comprimé pharmaceutique ou nutraceutique selon les revendications 1 à 2 comprenant des triglycérides DHA et/ou EPA.

4. Comprimé pharmaceutique ou nutraceutique selon l'une quelconque des revendications précédentes, où ledit comprimé comprend un agent actif supplémentaire tel qu'un produit pharmaceutique, un produit nutraceutique, une vitamine, un minéral ou un autre composé de complément hygiénique.

5. Comprimé pharmaceutique ou nutraceutique selon la revendication 4, dans lequel l'agent actif est sélectionné parmi un sel de calcium, de la simvastatine, de l'atorvastatine, de la glucosamine, au moins une vitamine et de l'acide folique.

6. Comprimé pharmaceutique ou nutraceutique selon l'une quelconque des revendications précédentes, dans lequel ledit au moins un acide gras comprend un acide gras oméga-3.

7. Comprimé pharmaceutique ou nutraceutique selon l'une quelconque des revendications précédentes, dans lequel ledit au moins un acide gras comprend un acide gras oméga-3 et au moins un acide gras oméga-6.

8. Comprimé pharmaceutique ou nutraceutique selon l'une quelconque des revendications précédentes, préparé par compression directe.

9. Comprimé pharmaceutique ou nutraceutique selon la revendication 5, comprenant du carbonate de calcium.

10. Procédé pour la préparation d'un comprimé pharmaceutique ou nutraceutique décrit dans l'une quelconque des revendications 1 à 9 comprenant la compression directe d'au moins 20 % en poids de complexe solide formé entre au moins deux triglycérides d'acide gras et au moins une cyclodextrine, dans lequel la teneur desdits au moins deux triglycérides d'acide gras dans ledit complexe est 10 % en poids ou plus, dans lequel le rapport pondéral de composés d'acide gras (total) sur la cyclodextrine est 1:5 à 5:1, et dans lequel la cyclodextrine est une bêta-cyclodextrine.

11. Comprimé pharmaceutique selon la revendication 1 à 9 à utiliser dans le traitement et le traitement prophylactique de maladies du système cardiovasculaire et osseux.
